# EUROPEAN PATENT SPECIFICATION

(11) **EP 4 442 733 B1**
(45) Date of publication and mention of the grant of the patent: **10.09.2025**
(21) Application number: 23739955.5
(22) Date of filing: 09.01.2023
(51) Int. Cl.: C08J 3/075, C08J 3/24, C08G 59/04, A61L 27/20, A61L 27/50, C08L 5/08, A61K 8/73, A61K 8/86, A61P 17/00, A61P 17/18, A61Q 19/00, A61Q 19/08, C08G 81/00, A61K 31/728, C08B 37/08, C08G 65/26

(54) **HYALURONIC ACID DERIVATIVE OR SALT THEREOF, AND PREPARATION METHOD THEREFOR AND APPLICATION THEREOF**
HYALURONSÄUREDERIVAT ODER SALZ DAVON SOWIE HERSTELLUNGSVERFAHREN DAFÜR UND ANWENDUNG DAVON
DÉRIVÉ D'ACIDE HYALURONIQUE OU SEL DE CELUI-CI, SON PROCÉDÉ DE PRÉPARATION ET SON APPLICATION

(30) Priority: 14.01.2022 CN 202210042774
(43) Date of publication of application: 09.10.2024
(73) Proprietor: Jenkem Technology Co., Ltd. (Tianjin), Tianjin 300462 (CN)
(72) Inventor: LIN, Meina, Tianjin 300462 (CN); CHEN, Xiaomeng, Tianjin 300462 (CN); SUN, Yu, Tianjin 300462 (CN); ZHAO, Xuan, Tianjin 300462 (CN)
(74) Representative: HGF
(86) International application number: PCT/CN2023/071208
(87) International publication number: WO 2023/134613

(56) References cited:
- EP-A1- 2 644 623
- CN-A- 103 360 633
- CN-A- 107 880 282
- CN-A- 109 096 483
- CN-A- 111 423 591
- US-A1- 2020 147 262
- KAZUTERU ET AL: "Hyaluronic acid grafted with poly(ethylene glycol) as a novel peptide formulation - ScienceDirect", vol. 59, no. 1, 1 May 1999 (1999-05-01), NL, pages 77 - 86, XP093253682, ISSN: 0168-3659, Retrieved from the Internet <URL:https://www.sciencedirect.com/science/article/pii/S0168365998001837?via=ihub#FIG2> [retrieved on 20250224], DOI: 10.1016/S0168-3659(98)00183-7

## Description

### TECHNICAL FIELD

The present invention relates to the technical field of macromolecules, and particularly relates to a hyaluronic acid derivative or a salt thereof, and a preparation method therefor and use thereof.

### BACKGROUND

Hyaluronic acid (HA) is a naturally occurring biopolymer material, which widely exists in connective tissues such as synovial fluid, eyeball vitreous body, skin, intercellular space, and cockscomb of animals and humans, is an important component constituting synovial fluid, vitreous body, skin, and cartilage tissue, and has unique physicochemical properties and wide biological functions. Hyaluronic acid is popular with beauty lovers in the cosmetic field because of its good biocompatibility, unique viscoelasticity, and excellent moisture-retaining and water-locking properties, and currently can be used for injecting into facial skin, reducing wrinkles, improving skin appearance and preventing skin aging.

Hyaluronic acid mostly exists in the form of sodium hyaluronate during production and application. Natural sodium hyaluronate is easily biodegradable, has short residence time in organisms, and has insufficient hardness and mechanical strength, so that the application thereof is limited. Therefore, sodium hyaluronate is usually prepared into a gel by using a crosslinking agent, and a sodium hyaluronate cross-linked modified gel with significantly improved enzymolysis resistance is developed on the basis of keeping the original biocompatibility, so that the application thereof is wider. However, any residual of crosslinking agent cannot be completely removed under the prior art, and the crosslinking agents are mostly chemical reagents, so that the excessive crosslinking agent may lead to the reduction of biocompatibility. When the crosslinking agent is injected into a human body, it is easy to cause immune reaction, is irritating to the skin and may even be cytotoxic.

In the research of non-cross-linked sodium hyaluronate, the documents, Adrian Ranga, etc. Hyaluronic acid hydrogels formed in situ by transglutaminase-catalyzed reaction[J]. Biomacromolecules and Kazuteru Moriyama, etc. Hyaluronic acid grafted with poly(ethylene glycol) as a novel peptide formulation. Journal of Controlled Release, reported the use of carboxyl in a sodium hyaluronate molecule for reaction to link polyethylene glycol.

The document, Irina M. Le-Deygen, etc. Poly(Ethylene Glycol) Interacts with Hyaluronan in Aqueous Media. Biomacromolecules, reported the use of hydroxyl in a sodium hyaluronate molecule for hydrogen bonding with polyethylene glycol to link polyethylene glycol.

In the prior art, amino, carboxyl, and hydroxyl on a sodium hyaluronate molecule are reacted with polyethylene glycol to prepare PEGylated sodium hyaluronates with different performances. However, there is currently no report on preparing a PEGylated sodium hyaluronate derivative by modifying a sodium hyaluronate molecule in an ether bond manner through the reaction between hydroxyl in a sodium hyaluronate molecule and a methoxy polyethylene glycol epoxide derivative.

### SUMMARY

The present invention overcomes the defects of the prior art. In a first aspect, the present invention provides a derivative of hyaluronic acid or a salt thereof, and the derivative of hyaluronic acid has a structure of formula (I): 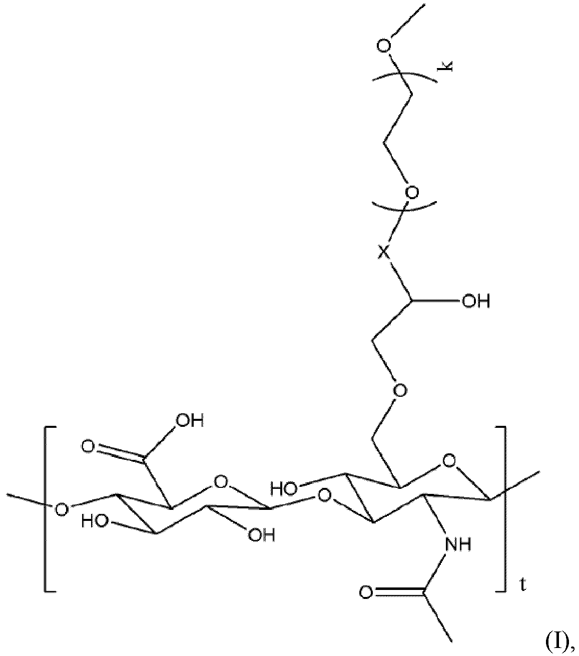
wherein X is selected from: one or a combination of two or more of -(CR₁R₂)ₘ-, -(CH₂)ₘNH-, -NHCO(CH₂)ₘ-, -(CH₂)ₘCONH-, and -CO(CH₂)ₘ-, wherein m is an integer of 1-10;
R₁ and R₂ are independently selected from: H, alkyl, cycloalkyl, cycloalkylalkyl, alkenyl, aryl, aralkyl, heterocyclyl, heterocyclylalkyl, -COR₃, -C(O)OR₃, -C(O)NR₃R₄, -CH=NR₃, -CN, - OR₃, -OC(O)R₃, -S(O)ₙ-R₃, -NR₃R₄, -NR₃C(O)R₄, and halogen;
n is selected from 0, 1, and 2;
R₃ and R₄ are independently selected from hydrogen, alkyl, cycloalkyl, alkenyl, aryl, heterocyclyl, and halogen;
k is the degree of polymerization of the polyethylene glycol residue, and k is an integer of 40-4600 (e.g., 40, 41, 42, 43, 45, 46, 47, 48, 49, 50, 55, 60, 65, 70, 75, 80, 85, 90, 95, 100, 105, 110, 111, 112, 113, 114, 115, 116, 117, 118, 119, 120, 150, 200, 400, 500, 800, 1000, 2000, 3000, 4000, and 4500); preferably, k is an integer of 45-4545; more preferably, k is an integer of 75-4545; particularly preferably, k is an integer of 100-4545;
t is the degree of polymerization of the hyaluronic acid residue, and t is an integer of 100-8000 (e.g., 100, 105, 110, 115, 120, 125, 130, 135, 140, 145, 150, 200, 300, 400, 500, 1000, 2000, 3000, 4000, 5000, 5500, 6000, 5500, 7000, 7100, 7200, 7300, and 7400); preferably, t is an integer of 120-7500; more preferably, t is an integer of 700-6000; particularly preferably, t is an integer of 1000-4000;
preferably, X is -(CR₁R₂)ₘ-;
preferably, m is an integer of 1-5, such as 1, 2, 3, 4, or 5;
in one embodiment of the present invention, m = 1.

Preferably, R₁ and R₂ are independently selected from: H, C₁-C₆ alkyl, C₃-C₆ cycloalkyl, -OR₃, -NR₃R₄, and halogen.

Preferably, R₃ and R₄ are independently selected from: hydrogen and C₁-C₃ alkyl.

In one embodiment of the present invention, R₁ and R₂ are both H.

In a preferred embodiment of the present invention, X is -CH₂-.

The salt is selected from: one or more of sodium salts, potassium salts, calcium salts, magnesium salts, zinc salts, cobalt salts, and tetrabutylammonium salts; preferably, the salt is a sodium salt.

In one embodiment of the present invention, the hyaluronic acid derivative has the following structure:

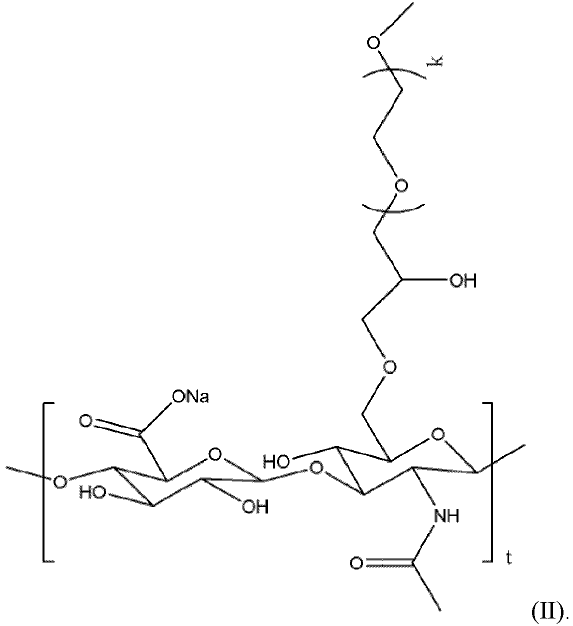

In a second aspect of the present invention, provided is a preparation method for a hyaluronic acid derivative of general formula (II) or a salt thereof, wherein the preparation method comprises the following steps:
(1) weighing out hyaluronic acid, mixing the hyaluronic acid with an alkaline solvent, and stirring;
(2) adding a methoxy polyethylene glycol epoxide derivative into the solution obtained in step (1) for reaction,
wherein the methoxy polyethylene glycol epoxide derivative has a structure of formula (III):

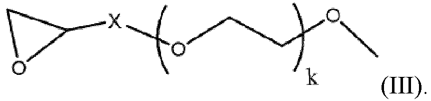

Preferably, the alkaline solvent in step (1) is: one or more of sodium hydroxide, potassium hydroxide, sodium carbonate, potassium carbonate, sodium bicarbonate, and potassium bicarbonate.

In a preferred embodiment of the present invention, the alkaline solvent is a sodium hydroxide solution at a mass concentration of 0.25%-0.8% (such as 0.25%, 0.35%, 0.5%, and 0.8%), preferably a sodium hydroxide solution at a mass concentration of 0.35%-0.5%.

The sodium hyaluronate has a molecular weight of 40000-3200000 daltons; preferably, the sodium hyaluronate has a molecular weight of 50000-3000000 daltons; more preferably, the sodium hyaluronate has a molecular weight of 280000-2400000 daltons; particularly preferably, the sodium hyaluronate has a molecular weight of 400000-1600000 daltons.

The mass ratio of the hyaluronic acid to the alkaline solvent is in the range of 1:5 to 1:30 (specifically such as 1:5, 1:10, 1:15, 1:20, 1:25, or 1:30); preferably, the mass ratio of the hyaluronic acid to the alkaline solvent is 1:10.

The molar ratio of the hyaluronic acid to polymer units in the methoxy polyethylene glycol epoxide derivative is in the range of 0.1:1 to 10:1 (specifically such as 0.1:1, 0.2:1, 0.3:1, 0.4:1, 0.5:1, 0.6:1, 0.7:1, 0.8:1, 0.9:1, 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1, and 10:1).

The stirring in step (1) is performed for a period of 5-50 min, preferably 10-30 min (such as 15 min, 16 min, 17 min, 18 min, 19 min, and 20 min), and more preferably 10-20 min.

The polyethylene glycol epoxide derivative in step (2) is monomethoxy polyethylene glycol ethylene oxide; preferably, the monomethoxy polyethylene glycol ethylene oxide has a molecular weight of 2000-200000, e.g., 2000, 3000, 4000, 5000, 10000, 20000, 30000, 50000, and 100000.

The reaction in step (2) comprises stirring, mixing and letting stand.

Preferably, the reaction time in step (2) is 10-24 h, preferably 13-21 h, and more preferably 17-19 h.

Preferably, the preparation method for a hyaluronic acid derivative or a salt thereof further comprises a step of purifying the product, wherein the step of purifying the product comprises a step of adding an acidic reagent to adjust the solution obtained in step (2) to be neutral, and/or a step of adding a precipitant to precipitate the product, and/or a step of adding a detergent to wash the precipitate.

The acidic reagent is selected from: one or more of a dilute hydrochloric acid solution, a dilute sulfuric acid solution, a dilute acetic acid solution, and a dilute hypochlorous acid solution.

In one embodiment of the present invention, the acidic reagent is a dilute hydrochloric acid solution.

The precipitant is acetonitrile or ethanol.

In one embodiment of the present invention, the precipitant is acetonitrile.

The detergent is ethanol or water.

In one embodiment of the present invention, the detergent is ethanol.

In one embodiment of the present invention, the preparation method for a hyaluronic acid derivative or a salt thereof is weighing out hyaluronic acid, dissolving the hyaluronic acid with an alkaline solvent, stirring for 10-30 min until the mixture is uniform, then adding a methoxy polyethylene glycol epoxide derivative, stirring for another 10-24 h until the materials are uniformly mixed, letting stand at room temperature for 15-18 h, adding an acidic reagent to adjust the pH of the system to be neutral, then adding a precipitant for precipitation, vigorously stirring to make the product particles uniform, centrifuging and letting stand to remove the supernatant, performing precipitation for another 2-5 times with a precipitant, subsequently washing the obtained precipitate for 1-3 times with a detergent, and finally placing the obtained precipitation product in a vacuum drying oven for drying.

In a third aspect of the present invention, provided is use of the hyaluronic acid derivative of general formula (I) or (II) or a salt thereof in preparing a product for medicament, medical cosmetology, and cosmetic use.

Preferably, the use is use of the hyaluronic acid derivative of general formula (I) or (II) or a salt thereof in preparing a product for medical cosmetology use.

Preferably, the product for medical cosmetology use is a soft tissue filler.

The present invention further provides a soft tissue filler, which comprises the hyaluronic acid derivative of general formula (I) or (II) or a salt thereof.

According to the present invention, a polyethylene glycol derivative and hyaluronic acid or a salt thereof are modified in an ether bond manner, the reaction product is not in a cross-linked gel state but a composite polymer material which is soluble in an aqueous solution. The prepared hyaluronic acid derivative or a salt thereof not only maintains the water-locking and moisture-retaining performance of hyaluronic acid, but also enhances the hyaluronidase degradation resistance *in vivo.* When the prepared hyaluronic acid derivative or a salt thereof is applied to repair fine wrinkles on the face and neck, the injection frequency can be reduced, which enhances the comfort for the experiencer, and reduces the economic cost.

### BRIEF DESCRIPTION OF THE DRAWINGS

FIG. 1 shows the relationship between the enzymolysis molecular weight of the control sample and time.
FIG. 2 shows the relationship between the enzymolysis molecular weight of sample 8 and time.

### DETAILED DESCRIPTION

Unless otherwise defined, all scientific and technical terms used herein have the same meaning as commonly understood by those skilled in the art to which the present invention relates. For example: "alkyl" refers to a hydrocarbon chain radical that is linear or branched and that does not contain unsaturated bonds, and C₁-C₆ alkyl in the present invention refers to alkyl containing 1-6 carbon atoms, such as methyl, ethyl, *n*-propyl, isopropyl, *n*-butyl, isobutyl, *tert-*butyl, *n*-pentyl, and *n*-hexyl, preferably C₁-C₃ alkyl (such as methyl, ethyl, *n*-propyl, and isopropyl); "cycloalkyl" refers to an alicyclic hydrocarbon, typical cycloalkyl contains 1 to 4 monocyclic and/or fused rings and contains 3 to about 18 carbon atoms, and C₃-C₆ cycloalkyl in the present invention refers to cycloalkyl containing 3-6 carbon atoms, such as cyclopropyl, cyclopentyl, and cyclohexyl.

The technical solutions in the examples of the present invention will be described clearly and completely below, and it is apparent that the described examples are only a part of the examples of the present invention, but not all of them.

### Example 1

380 mg of HA 600000 was weighed out and dissolved in 3.8 mL of an aqueous 0.25% sodium hydroxide solution, and the mixture was vigorously stirred for 10-30 min until the mixture was uniform. 1 g of M-PEG2000-EPOX 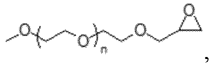 provided by JenKem Technology Co., Ltd., the same applies hereinafter) was added, and the mixture was then vigorously stirred until the materials were uniformly mixed, and left to stand at room temperature for 18 h. The next day, 1 mL of 0.25 N HCl was added to adjust the pH of the system to about 7, then 20 mL of acetonitrile was added for precipitation, and the mixture was vigorously stirred to make the product particles uniform, centrifuged and left to stand to remove the supernatant; precipitation was performed 4 times with 20 mL of acetonitrile, subsequently the precipitate was washed 2 times with 20 mL of absolute ethanol, and the product was placed in a vacuum drying oven for drying to give sample 1 (0.25% NaOH). Samples were taken to perform multiangle light-differential detection and nuclear magnetic resonance detection.

### Example 2

570 mg of HA 600000 was weighed out and dissolved in 5.7 mL of an aqueous 0.35% sodium hydroxide solution, and the mixture was vigorously stirred for 10-30 min until the mixture was uniform. 1.5 g of M-PEG2000-EPOX was added, and the mixture was then vigorously stirred until the materials were uniformly mixed, and left to stand at room temperature for 18 h. The next day, 1.5 mL of 0.35 N HCl was added to adjust the pH of the system to about 7, then 30 mL of acetonitrile was added for precipitation, and the mixture was vigorously stirred to make the product particles uniform, centrifuged and left to stand to remove the supernatant; precipitation was performed 4 times with 30 mL of acetonitrile, subsequently the precipitate was washed 2 times with 30 mL of absolute ethanol, and the product was placed in a vacuum drying oven for drying to give sample 2 (0.35% NaOH). Samples were taken to perform multiangle light-differential detection and nuclear magnetic resonance detection.

### Example 3

380 mg of HA 600000 was weighed out and dissolved in 3.8 mL of an aqueous 0.5% sodium hydroxide solution, and the mixture was vigorously stirred for 10-30 min until the mixture was uniform. 1 g of M-PEG2000-EPOX was added, and the mixture was then vigorously stirred until the materials were uniformly mixed, and left to stand at room temperature for 18 h. The next day, 1 mL of 0.5 N HCl was added to adjust the pH of the system to about 7, then 20 mL of acetonitrile was added for precipitation, and the mixture was vigorously stirred to make the product particles uniform, centrifuged and left to stand to remove the supernatant; precipitation was performed 4 times with 20 mL of acetonitrile, subsequently the precipitate was washed 2 times with 20 mL of absolute ethanol, and the product was placed in a vacuum drying oven for drying to give sample 3 (0.5% NaOH). Samples were taken to perform multiangle light-differential detection and nuclear magnetic resonance detection.

### Example 4

380 mg of HA 600000 was weighed out and dissolved in 3.8 mL of an aqueous 0.8% sodium hydroxide solution, and the mixture was vigorously stirred for 10-30 min until the mixture was uniform. 1 g of M-PEG2000-EPOX was added, and the mixture was then vigorously stirred until the materials were uniformly mixed, and left to stand at room temperature for 18 h. The next day, 1 mL of 0.8 N HCl was added to adjust the pH of the system to about 7, then 20 mL of acetonitrile was added for precipitation, and the mixture was vigorously stirred to make the product particles uniform, centrifuged and left to stand to remove the supernatant; precipitation was performed 4 times with 20 mL of acetonitrile, subsequently the precipitate was washed 2 times with 20 mL of absolute ethanol, and the product was placed in a vacuum drying oven for drying to give sample 4 (0.8% NaOH). Samples were taken to perform multiangle light-differential detection and nuclear magnetic resonance detection.

### Example 5

190 mg of HA 600000 was weighed out and dissolved in 5.7 mL of an aqueous 0.35% sodium hydroxide solution, and the mixture was vigorously stirred for 10-30 min until the mixture was uniform. 500 mg of M-PEG2000-EPOX was added, and the mixture was then vigorously stirred until the materials were uniformly mixed, and left to stand at room temperature for 18 h. The next day, 1 mL of 0.35 N HCl was added to adjust the pH of the system to about 7, then 10 mL of acetonitrile was added for precipitation, and the mixture was vigorously stirred to make the product particles uniform, centrifuged and left to stand to remove the supernatant; precipitation was performed 4 times with 10 mL of acetonitrile, subsequently the precipitate was washed 2 times with 10 mL of absolute ethanol, and the product was placed in a vacuum drying oven for drying to give sample 5 (0.35% NaOH). Samples were taken to perform multiangle light-differential detection and nuclear magnetic resonance detection.

### Example 6

760 mg of HA 600000 was weighed out and dissolved in 7.6 mL of an aqueous 0.35% sodium hydroxide solution, and the mixture was vigorously stirred for 10-30 min until the mixture was uniform. 2 g of M-PEG2000-EPOX was added, and the mixture was then vigorously stirred until the materials were uniformly mixed, and left to stand at room temperature for 15 h. The next day, 2 mL of 0.35 N HCl was added to adjust the pH of the system to about 7, then 40 mL of acetonitrile was added for precipitation, and the mixture was vigorously stirred to make the product particles uniform, centrifuged and left to stand to remove the supernatant; precipitation was performed 4 times with 40 mL of acetonitrile, subsequently the precipitate was washed 2 times with 40 mL of absolute ethanol, and the product was placed in a vacuum drying oven for drying to give sample 6 (0.35% NaOH). Samples were taken to perform multiangle light-differential detection and nuclear magnetic resonance detection.

### Example 7

760 mg of HA 600000 was weighed out and dissolved in 7.6 mL of an aqueous 0.35% sodium hydroxide solution, and the mixture was vigorously stirred for 10-30 min until the mixture was uniform. 2 g of M-PEG2000-EPOX was added, and the mixture was then vigorously stirred until the materials were uniformly mixed, and left to stand at room temperature for 21 h. The next day, 2 mL of 0.35 N HCl was added to adjust the pH of the system to about 7, then 40 mL of acetonitrile was added for precipitation, and the mixture was vigorously stirred to make the product particles uniform, centrifuged and left to stand to remove the supernatant; precipitation was performed 4 times with 40 mL of acetonitrile, subsequently the precipitate was washed 2 times with 40 mL of absolute ethanol, and the product was placed in a vacuum drying oven for drying to give sample 6 (0.35% NaOH). Samples were taken to perform multiangle light-differential detection and nuclear magnetic resonance detection.

### Example 8

570 mg of HA 600000 was weighed out and dissolved in 5.7 mL of an aqueous 0.35% sodium hydroxide solution, and the mixture was vigorously stirred for 10-30 min until the mixture was uniform. 2750 mg of M-PEG5000-EPOX was added, and the mixture was then vigorously stirred until the materials were uniformly mixed, and left to stand at room temperature for 18 h. The next day, 2 mL of 0.35 N HCl was added to adjust the pH of the system to about 7, then 30 mL of acetonitrile was added for precipitation, and the mixture was vigorously stirred to make the product particles uniform, centrifuged and left to stand to remove the supernatant; precipitation was performed 4 times with 30 mL of acetonitrile, subsequently the precipitate was washed 2 times with 30 mL of absolute ethanol, and the product was placed in a vacuum drying oven for drying to give sample 6 (0.35% NaOH). Samples were taken to perform multiangle light-differential detection and nuclear magnetic resonance detection.

### Example 9

570 mg of HA 600000 was weighed out and dissolved in 5.7 mL of an aqueous 0.35% sodium hydroxide solution, and the mixture was vigorously stirred for 10-30 min until the mixture was uniform. 1875 mg of M-PEG5000-EPOX was added, and the mixture was then vigorously stirred until the materials were uniformly mixed, and left to stand at room temperature for 18 h. The next day, 2 mL of 0.35 N HCl was added to adjust the pH of the system to about 7, then 30 mL of acetonitrile was added for precipitation, and the mixture was vigorously stirred to make the product particles uniform, centrifuged and left to stand to remove the supernatant; precipitation was performed 4 times with 30 mL of acetonitrile, subsequently the precipitate was washed 2 times with 30 mL of absolute ethanol, and the product was placed in a vacuum drying oven for drying to give sample 6 (0.35% NaOH). Samples were taken to perform multiangle light-differential detection and nuclear magnetic resonance detection.

### Example 10

570 mg of HA 600000 was weighed out and dissolved in 5.7 mL of an aqueous 0.35% sodium hydroxide solution, and the mixture was vigorously stirred for 10-30 min until the mixture was uniform. 7500 mg of M-PEG5000-EPOX was added, and the mixture was then vigorously stirred until the materials were uniformly mixed, and left to stand at room temperature for 18 h. The next day, 2 mL of 0.35 N HCl was added to adjust the pH of the system to about 7, then 30 mL of acetonitrile was added for precipitation, and the mixture was vigorously stirred to make the product particles uniform, centrifuged and left to stand to remove the supernatant; precipitation was performed 4 times with 30 mL of acetonitrile, subsequently the precipitate was washed 2 times with 30 mL of absolute ethanol, and the product was placed in a vacuum drying oven for drying to give sample 6 (0.35% NaOH). Samples were taken to perform multiangle light-differential detection and nuclear magnetic resonance detection.

### Example 11

570 mg of HA 600000 was weighed out and dissolved in 5.7 mL of an aqueous 0.35% sodium hydroxide solution, and the mixture was vigorously stirred for 10-30 min until the mixture was uniform, and left to stand at room temperature for 18 h. The next day, 2 mL of 0.35 N HCl was added to adjust the pH of the system to about 7, then 30 mL of acetonitrile was added for precipitation, and the mixture was vigorously stirred to make the product particles uniform, centrifuged and left to stand to remove the supernatant; precipitation was performed 4 times with 30 mL of acetonitrile, subsequently the precipitate was washed 2 times with 30 mL of absolute ethanol, and the product was placed in a vacuum drying oven for drying to give sample 11 (0.35% NaOH). Samples were taken to perform multiangle light-differential detection and nuclear magnetic resonance detection. The molecular weight was 6.366 × 10⁵ Da as detected by the multiangle light, and sample 11 was used as a comparative sample for calculating the number of linked polyethylene glycols on the sodium hyaluronate backbone.

### Example 12

The products of Examples 1-4 were subjected to multiangle light-differential detection, and the obtained molecular weight information is shown in Table 1:

**Table 1**

| Sample No. | Sample 1 (0.25%) | Sample 2 (0.35%) | Sample 3 (0.5%) | Sample 4 (0.8%) |
|---|---|---|---|---|
| Molecular weight (Mw × 10⁵) | 3.585 | 12.02 | 10.5 | 2.696 |

As can be seen from the molecular weight data in Table 1, the alkali concentration of the reaction solution should not be too low nor too high. If the concentration of the alkali liquor is too low, the reaction activities of the raw materials are not high; if the concentration of the alkali liquor is too high, more sodium hyaluronate raw material will undergo alkaline hydrolysis.

The products of Example 2 and Example 5 were subjected to multiangle light-differential detection, and the obtained molecular weight information is shown in Table 2:

**Table 2**

| Sample No. | NaOH concentration (%) | Solvent ratio (based on HA) mg/mL | Molecular weight (Mw × 10⁵) |
|---|---|---|---|
| Sample 2 (0.35%) | 0.35% | 10 | 12.02 |
| Sample 5 (0.35%) | 0.35% | 30 | 4.005 |

As can be seen from the molecular weight data in Table 2, too much solvent results in too dilute a system concentration and also accelerates the degree of alkaline hydrolysis of the raw material sodium hyaluronate.

The products of Example 2, Example 6, and Example 7 were subjected to multiangle light-differential detection, and the obtained molecular weight information is shown in Table 3:

**Table 3**

| Sample No. | NaOH concentration (%) | Reaction time (h) at room temperature | Molecular weight (Mw × 10⁵) |
|---|---|---|---|
| Sample 2 (0.35%) | 0.35% | 18 | 12.02 |
| Sample 6 (0.35%) | 0.35% | 15 | 9.352 |
| Sample 7 (0.35%) | 0.35% | 21 | 8.693 |

As can be seen from the molecular weight data in Table 3, the reaction time is also a factor limiting the product performance, and too long a reaction time increases the risk of degradation of the sodium hyaluronate material.

The products of Example 8, Example 9, and Example 10 were subjected to multiangle light-differential detection, and the obtained molecular weight information is shown in Table 4:

**Table 4**

| Sample No. | NaOH concentration (%) | HA:M-PEG5000-EPOX mol ratio | Molecular weight (Mw × 10⁵) |
|---|---|---|---|
| Sample 8 (0.35%) | 0.35% | 2:1 | 12.82 |
| Sample 9 (0.35%) | 0.35% | 4:1 | 10.2 |
| Sample 10 (0.35%) | 0.35% | 1:1 | 10.07 |

As can be seen from the molecular weight data in Table 4, the feeding ratio of HA to methoxy polyethylene glycol epoxide derivative also has some influence on the performance of the product.

### Example 13

Taking sample 2 and sample 11 as examples, the calculation method for the ratio of the number of sodium hyaluronate disaccharides in the PEGylated sodium hyaluronate product to the number of PEG is as follows (the molecular weight of one disaccharide unit is 380).
The average number of disaccharide units in sample 11 was: 6.366 × 105/380 = 1675;
the number of PEG2000 contained in sample 2 was: (12.02-6.366) 105/2000 = 283
the ratio of the number of disaccharides to the number of PEG2000 in sample 2 was: 1675/283 = 6:1.

*In vitro* enzymolysis experiment:
The enzymolysis experiment operation steps: Taking sample 8 as an example, 3.4 mg of sample 8 was weighed out, 242 µL of sterile water for injection was added, and the mixture was vortexed for 20 min. 242 µL of an aqueous sterile solution for injection containing 10 U/mL hyaluronidase was added, and the sample was mixed well and placed in a constant-temperature water bath environment at 37 °C. Samples were taken every 1 h, and the change of the molecular weight was tested using a multiangle light-differential detection method.

The sample information of the test enzymolysis experiment was as follows.

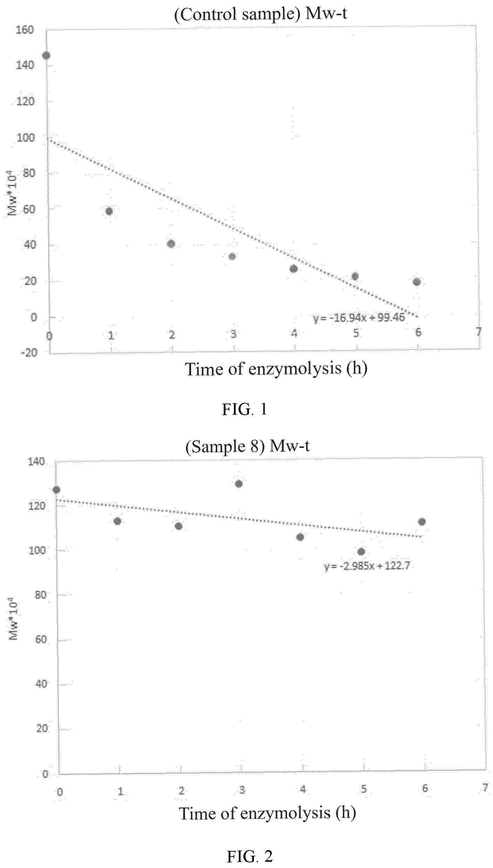

Control sample: HA 1200000 Da, the relationship between the enzymolysis molecular weight and time is shown in FIG. 1.

Sample 2: The ratio of the number of HA disaccharides to the number of PEG2000 was 6: 1.

Sample 12: The ratio of the number of HA disaccharides to the number of PEG2000 was 17:1.

Sample 8: The ratio of the number of HA disaccharides to the number of PEG5000 was 13:1, and the relationship between the enzymolysis molecular weight and time is shown in FIG. 2.

Sample 9: The ratio of the number of HA disaccharides to the number of PEG5000 was 22: 1.

Sample 13: The ratio of the number of HA disaccharides to the number of PEG5000 was 42:1.

Enzymolysis data of PEG5000-HA sample:

**Table 5**

| | 1h (×10⁴) | 2h (×10⁴) | 3h (×10⁴) | 4h (×10⁴) | 5h (×10⁴) | 6h (×10⁴) |
|---|---|---|---|---|---|---|
| HA 1200000 Da | 57.1 | 35.9 | 32.6 | 29 | 24.9 | 7.7 |
| Sample 8 (13:1) | 113.3 | 110.8 | 129.3 | 105.3 | 98.4 | 111.6 |
| Sample 9 (22:1) | 92 | 90.2 | 83.5 | | | |
| Sample 13 (42:1) | 54.3 | 51.2 | | | | |

**Table 6**

| Enzymolysis data of PEG2000-HA sample: | | | | | | |
|---|---|---|---|---|---|---|
| | 1h (×10⁴) | 2h (×10⁴) | 3h (×10⁴) | 4h (×10⁴) | 5h (×10⁴) | 6h (×10⁴) |
| HA 1200000 Da | 57.1 | 35.9 | 32.6 | 29 | 24.9 | 7.7 |
| Sample 2 (6:1) | 87.3 | 50.5 | 48.6 | 49 | 48 | 52.6 |
| Sample 12 (17:1) | 27.6 | 19.2 | 17.4 | | | |

As can be seen from the data in Table 5 and Table 6, when the molecular weight of PEG was 5000 and the ratio of HA to PEG was 13:1 to 42:1, the PEG has an effect on the enzyme degradation resistance of HA, and the higher the ratio was, the more significant the effect was; when the molecular weight of PEG was 2000, the effect of PEG on the enzyme degradation resistance of HA was not so great.

## Claims

1. A hyaluronic acid derivative or a salt thereof, wherein the hyaluronic acid derivative has a structure of formula (I): 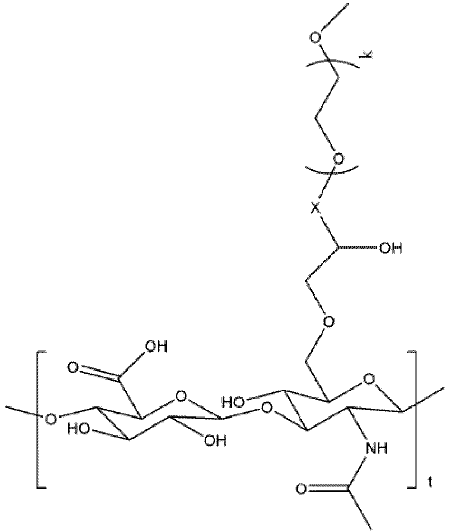
wherein X is selected from: one or a combination of two or more of -(CR₁R₂)ₘ-, -(CH₂)ₘNH-, -NHCO(CH₂)ₘ-, -(CH₂)ₘCONH-, and -CO(CH₂)ₘ-, wherein m is an integer of 1-10;
R₁ and R₂ are independently selected from: H, alkyl, cycloalkyl, cycloalkylalkyl, alkenyl, aryl, aralkyl, heterocyclyl, heterocyclylalkyl, -COR₃, -C(O)OR₃, -C(O)NR₃R₄, -CH=NR₃, -CN, - OR₃, -OC(O)R₃, -S(O)ₙ-R₃, -NR₃R₄, -NR₃C(O)R₄, and halogen;
n is selected from 0, 1, and 2;
R₃ and R₄ are independently selected from hydrogen, alkyl, cycloalkyl, alkenyl, aryl, heterocyclyl, and halogen;
k is the degree of polymerization of the polyethylene glycol residue, and k is an integer of 40-4600;
t is the degree of polymerization of the hyaluronic acid residue, and t is an integer of 100-8000.

2. The hyaluronic acid derivative or the salt thereof according to claim 1, wherein k is an integer of 45-4545; preferably, k is an integer of 75-4545; more preferably, k is an integer of 100-4545;
t is an integer of 120-7500; preferably, t is an integer of 700-6000; more preferably, t is an integer of 1000-4000.

3. The hyaluronic acid derivative or the salt thereof according to claim 1, wherein X is - (CR₁R₂)ₘ-, and m is an integer of 1-5; preferably, m is 1.

4. The hyaluronic acid derivative or the salt thereof according to claim 1, wherein R₁ and R₂ are independently selected from: H, C₁-C₆ alkyl, C₃-C₆ cycloalkyl, -OR₃, -NR₃R₄, and halogen, and R₃ and R₄ are independently selected from: hydrogen and C₁-C₃ alkyl.

5. The hyaluronic acid derivative or the salt thereof according to claim 1, wherein R₁ and R₂ are both H.

6. The hyaluronic acid derivative or the salt thereof according to claim 1, wherein the salt is selected from: one or more of sodium salts, potassium salts, calcium salts, magnesium salts, zinc salts, cobalt salts, and tetrabutylammonium salts; preferably, the salt is a sodium salt having a structure of formula (II):

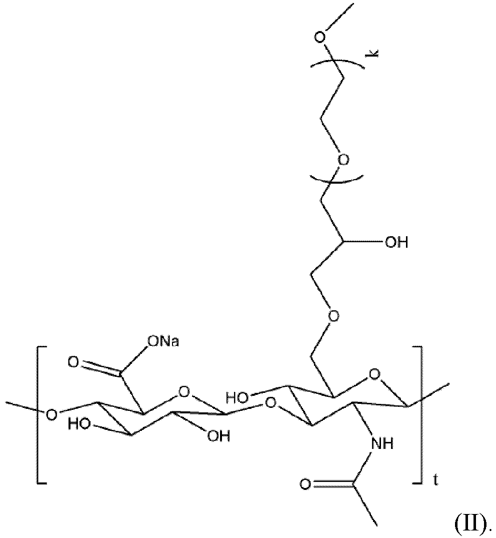

7. A preparation method for the hyaluronic acid derivative or the salt thereof according to claim 1, wherein the preparation method comprises the following steps:
(1) weighing out hyaluronic acid, mixing the hyaluronic acid with an alkaline solvent, and stirring;
(2) adding a methoxy polyethylene glycol epoxide derivative into the solution obtained in step (1) for reaction,
wherein the methoxy polyethylene glycol epoxide derivative has a structure of formula (III):

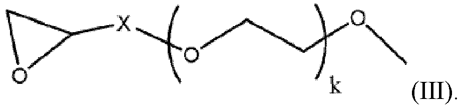

8. The preparation method for the hyaluronic acid derivative or the salt thereof according to claim 7, wherein the preparation method further comprises a step of purifying the product, and the step of purifying the product comprises a step of adding an acidic reagent to adjust the solution obtained in step (2) to be neutral, and/or a step of adding a precipitant to precipitate the product, and/or a step of adding a detergent to wash the precipitate.

9. The preparation method for the hyaluronic acid derivative or the salt thereof according to claim 7, wherein the mass ratio of the hyaluronic acid to the alkaline solvent is in the range of 1:5 to 1:30; preferably, the mass ratio of the hyaluronic acid to the alkaline solvent is 1:10.

10. The preparation method for the hyaluronic acid derivative or the salt thereof according to claim 7, wherein the molar ratio of the hyaluronic acid to polymer units in the methoxy polyethylene glycol epoxide derivative is in the range of 0.1:1 to 10:1.

11. The preparation method for the hyaluronic acid derivative or the salt thereof according to claim 7, wherein the reaction time in step (2) is 10-24 h, preferably 13-21 h, and more preferably 17-19 h.

12. Use of the hyaluronic acid derivative or the salt thereof according to claim 1 in preparing a product for medicament, medical cosmetology, and cosmetic use; preferably, the use is use of the hyaluronic acid derivative or the salt thereof described above in preparing a product for medical cosmetology use; more preferably, the product for medical cosmetology use is a soft tissue filler.

## Patentansprüche

1. Hyaluronsäurederivat oder ein Salz davon, wobei das Hyaluronsäurederivat eine Struktur der Formel (I) aufweist: 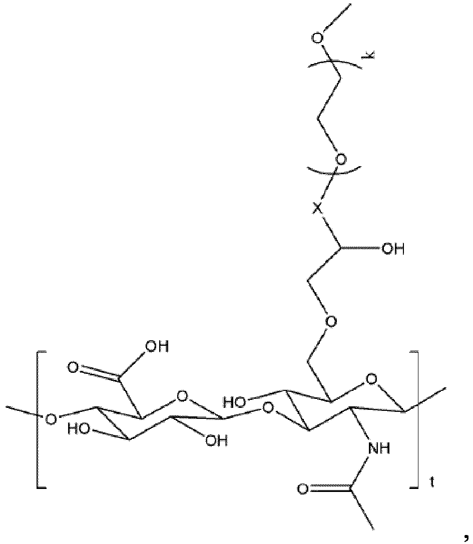
wobei X ausgewählt ist aus: einem oder einer Kombination von zwei oder mehr von - (CR₁R₂)ₘ-, -(CH₂)ₘNH-, -NHCO(CH₂)ₘ-, -(CH₂)ₘCONH- und -CO(CH₂)ₘ-, wobei m eine ganze Zahl von 1-10 ist;
R₁ und R₂ unabhängig ausgewählt sind aus: H, Alkyl, Cycloalkyl, Cycloalkylalkyl, Alkenyl, Aryl, Aralkyl, Heterocyclyl, Heterocyclylalkyl, -COR₃, -C(O)OR₃, -C(O)NR₃R₄, - CH=NR₃, -CN, -OR₃, -OC(O)R₃, -S(O)ₙ-R₃, -NR₃R₄, -NR₃C(O)R₄ und Halogen;
n ausgewählt ist aus 0, 1 und 2;
R₃ und R₄ unabhängig ausgewählt sind aus Wasserstoff, Alkyl, Cycloalkyl, Alkenyl, Aryl, Heterocyclyl und Halogen;
k der Polymerisationsgrad des Polyethylenglykolrestes ist und k eine ganze Zahl von 40-4600 ist;
t der Polymerisationsgrad des Hyaluronsäurerestes ist und t eine ganze Zahl von 100-8000 ist.

2. Hyaluronsäurederivat oder das Salz davon nach Anspruch 1, wobei k eine ganze Zahl von 45-4545 ist; bevorzugt k eine ganze Zahl von 75-4545 ist; bevorzugter k eine ganze Zahl von 100-4545 ist;
t eine ganze Zahl von 120-7500 ist; bevorzugt t eine ganze Zahl von 700-6000 ist; bevorzugter t eine ganze Zahl von 1000-4000 ist.

3. Hyaluronsäurederivat oder das Salz davon nach Anspruch 1, wobei X -(CR₁R₂)ₘ- ist und m eine ganze Zahl von 1-5 ist; bevorzugt m 1 ist.

4. Hyaluronsäurederivat oder das Salz davon nach Anspruch 1, wobei R₁ und R₂ unabhängig ausgewählt sind aus: H, C₁-C₆-Alkyl, C₃-C₆-Cycloalkyl, -OR₃, -NR₃R₄ und Halogen, und R₃ und R₄ unabhängig ausgewählt sind aus: Wasserstoff und C₁-C₃-Alkyl.

5. Hyaluronsäurederivat oder das Salz davon nach Anspruch 1, wobei R₁ und R₂ beide H sind.

6. Hyaluronsäurederivat oder das Salz davon nach Anspruch 1, wobei das Salz ausgewählt ist aus: einem oder mehreren von Natriumsalzen, Kaliumsalzen, Calciumsalzen, Magnesiumsalzen, Zinksalzen, Kobaltsalzen und Tetrabutylammoniumsalzen; bevorzugt das Salz ein Natriumsalz mit einer Struktur der Formel (II) ist:

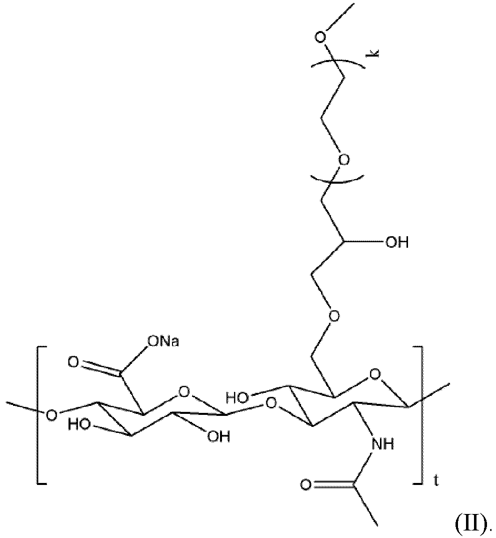

7. Herstellungsverfahren für das Hyaluronsäurederivat oder das Salz davon nach Anspruch 1, wobei das Herstellungsverfahren die folgenden Schritte umfasst:
(1) Abwiegen von Hyaluronsäure, Mischen der Hyaluronsäure mit einem alkalischen Lösungsmittel und Rühren;
(2) Hinzufügen eines Methoxy-Polyethylenglykolepoxid-Derivats in die Lösung, die in Schritt (1) erhalten wird, zur Reaktion,
wobei das Methoxy-Polyethylenglykolepoxid-Derivat eine Struktur der Formel (III) aufweist:

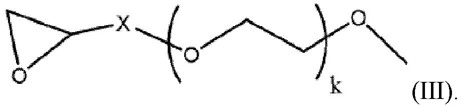

8. Herstellungsverfahren für das Hyaluronsäurederivat oder das Salz davon nach Anspruch 7, wobei das Herstellungsverfahren ferner einen Schritt des Reinigens des Produkts umfasst und der Schritt des Reinigens des Produkts einen Schritt des Hinzufügen eines sauren Reagens, um die Lösung, die in Schritt (2) erhalten wird, einzustellen, um neutral zu sein, und/oder einen Schritt des Hinzufügens eines Präzipitats, um das Produkt zu präzipitieren, und/oder einen Schritt des Hinzufügens eines Detergens, um das Präzipitat zu waschen, umfasst.

9. Herstellungsverfahren für das Hyaluronsäurederivat oder das Salz davon nach Anspruch 7, wobei das Massenverhältnis der Hyaluronsäure zu dem alkalischen Lösungsmittel in dem Bereich von 1:5 bis 1:30 ist; bevorzugt das Massenverhältnis der Hyaluronsäure zu dem alkalischen Lösungsmittel 1:10 ist.

10. Herstellungsverfahren für das Hyaluronsäurederivat oder das Salz davon nach Anspruch 7, wobei das Molverhältnis der Hyaluronsäure zu Polymereinheiten in dem Methoxy-Polyethylenglykolepoxid-Derivat in dem Bereich von 0,1:1 bis 10:1 ist.

11. Herstellungsverfahren für das Hyaluronsäurederivat oder das Salz davon nach Anspruch 7, wobei die Reaktionszeit in Schritt (2) 10-24 h, bevorzugt 13-21 h und bevorzugter 17-19 h ist.

12. Verwendung des Hyaluronsäurederivats oder des Salzes davon nach Anspruch 1 beim Herstellen eines Produkts für Medikamente, medizinische Kosmetik und kosmetische Verwendung; wobei bevorzugt die Verwendung Verwendung des oben beschriebenen Hyaluronsäurederivats oder des Salzes davon beim Herstellen eines Produkts für Verwendung in der medizinischen Kosmetik ist; bevorzugter das Produkt für Verwendung in der medizinischen Kosmetik ein Weichgewebefüller ist.

## Revendications

1. Dérivé d'acide hyaluronique ou sel de celui-ci, ledit dérivé d'acide hyaluronique présente une structure de formule (I) : 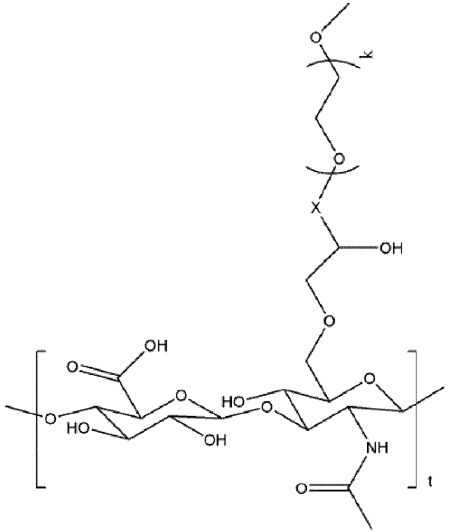
dans lequel X est choisi parmi : un ou une combinaison de deux ou plus parmi -(CR₁R₂)ₘ-, -(CH₂)ₘNH-, -NHCO(CH₂)ₘ-, -(CH₂)ₘCONH- et -CO(CH₂)ₘ-, dans lequel m est un entier de 1 à 10 ;
R₁ et R₂ sont choisis indépendamment parmi : H, un alkyle, un cycloalkyle, un cycloalkylalkyle, un alcényle, un aryle, un aralkyle, un hétérocyclyle, un hétérocyclylalkyle, - COR₃, -C(O)OR₃, -C(O)NR₃R₄, -CH=NR₃, -CN, -OR₃, -OC(O)R₃, -S(O)ₙ-R₃, -NR₃R₄, - NR₃C(O)R₄ et un halogène ;
n est choisi parmi 0, 1 et 2 ;
R₃ et R₄ sont indépendamment choisis parmi un hydrogène, un alkyle, un cycloalkyle, un alcényle, un aryle, un hétérocyclyle et un halogène ;
k est le degré de polymérisation du résidu de polyéthylène glycol, et k est un entier de 40 à 4600 ;
t est le degré de polymérisation du résidu d'acide hyaluronique, et t est un entier de 100 à 8000.

2. Dérivé d'acide hyaluronique ou sel de celui-ci selon la revendication 1, dans lequel k est un entier de 45 à 4545 ; de préférence, k est un entier de 75 à 4545 ; idéalement, k est un entier de 100 à 4545 ;
t est un entier de 120 à 7500 ; de préférence, t est un entier de 700 à 6000 ; idéalement, t est un entier de 1000 à 4000.

3. Dérivé d'acide hyaluronique ou sel de celui-ci selon la revendication 1, dans lequel X est -(CR₁R₂)ₘ-, et m est un entier de 1 à 5 ; de préférence, m vaut 1.

4. Dérivé d'acide hyaluronique ou sel de celui-ci selon la revendication 1, dans lequel R₁ et R₂ sont choisis indépendamment parmi : H, un alkyle en C₁-C₆, un cycloalkyle en C₃-C₆, -OR₃, - NR₃R₄ et un halogène, et R₃ et R₄ sont indépendamment choisis parmi : un hydrogène et un alkyle en C₁-C₃.

5. Dérivé d'acide hyaluronique ou sel de celui-ci selon la revendication 1, dans lequel R₁ et R₂ sont tous deux H.

6. Dérivé d'acide hyaluronique ou sel de celui-ci selon la revendication 1, dans lequel le sel est choisi parmi : un ou plusieurs sels de sodium, sels de potassium, sels de calcium, sels de magnésium, sels de zinc, sels de cobalt et sels de tétrabutylammonium ; de préférence, le sel est un sel de sodium présentant une structure de formule (II) :

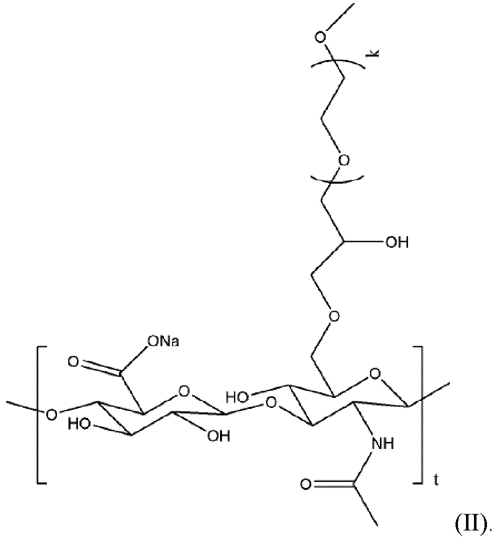

7. Procédé de préparation pour le dérivé d'acide hyaluronique ou du sel de celui-ci selon la revendication 1, dans lequel le procédé de préparation comprend les étapes suivantes :
(1) pesée de l'acide hyaluronique, mélange de l'acide hyaluronique avec un solvant alcalin, et agitation ;
(2) ajout d'un dérivé époxyde de méthoxy polyéthylène glycol à la solution obtenue à l'étape (1) pour la réaction,
dans lequel le dérivé époxyde de méthoxy polyéthylène glycol présente une structure de formule (III) :

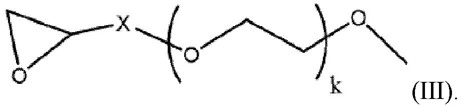

8. Procédé de préparation pour le dérivé d'acide hyaluronique ou du sel de celui-ci selon la revendication 7, ledit procédé de préparation comprenant en outre une étape de purification du produit, et l'étape de purification du produit comprend une étape d'ajout d'un réactif acide pour ajuster la solution obtenue à l'étape (2) pour qu'elle soit neutre, et/ou une étape d'ajout d'un précipitant pour précipiter le produit, et/ou une étape d'ajout d'un détergent pour laver le précipité.

9. Procédé de préparation pour le dérivé d'acide hyaluronique ou du sel de celui-ci selon la revendication 7, dans lequel le rapport massique entre l'acide hyaluronique et le solvant alcalin est dans la plage de 1:5 à 1:30 ; de préférence, le rapport massique entre l'acide hyaluronique et le solvant alcalin est de 1:10.

10. Procédé de préparation pour le dérivé d'acide hyaluronique ou du sel de celui-ci selon la revendication 7, dans lequel le rapport molaire entre l'acide hyaluronique et des unités polymères dans le dérivé époxyde de méthoxy polyéthylène glycol est dans la plage de 0,1:1 à 10:1.

11. Procédé de préparation pour le dérivé d'acide hyaluronique ou du sel de celui-ci selon la revendication 7, dans lequel le temps de réaction à l'étape (2) est de 10 à 24 h, de préférence de 13 à 21 h, et idéalement de 17 à 19 h.

12. Utilisation du dérivé d'acide hyaluronique ou du sel de celui-ci selon la revendication 1 dans la préparation d'un produit destiné à être utilisé en médecine, en cosmétologie médicale et en cosmétique ; de préférence, l'utilisation est l'utilisation du dérivé d'acide hyaluronique ou du sel de celui-ci décrit ci-dessus dans la préparation d'un produit destiné à être utilisé en cosmétologie médicale ; idéalement, le produit destiné à être utilisé en cosmétologie médicale est un produit de comblement pour tissus mous.
